# EUROPEAN PATENT APPLICATION

(11) **EP 4 226 973 A1**
(43) Date of publication of application: **16.08.2023**
(21) Application number: 22156290.3
(22) Date of filing: 11.02.2022
(51) Int. Cl.: A61P 35/04, G01N 33/573, A61P 37/02, C12N 9/16

(54) **METHOD OF PREVENTING AND/OR REDUCING METASTASIS OF TUMOR CELLS, PREVENTING OR REDUCING SENESCENCE, INHIBITING DIFFERENTIATION AND OF REDUCING INFLAMMATION**

(71) Applicant: Albert-Ludwigs-Universität Freiburg, 79098 Freiburg (DE)
(72) Inventor: HÄCKER, Georg, 79108 Freiburg (DE); HAIMOVICI, Aladin, 79100 Freiburg (DE); WEBER, Arnim, 79100 Freiburg (DE)
(74) Representative: Lederer & Keller Patentanwälte Partnerschaft mbB

(57) **Abstract**

The present application refers to a method of preventing or reducing metastasis of tumor cells, senescence, differentiation or inflammation wherein the caspase-activated DNAse which induces strand breaks in the genomic DNA of cells causing changes of the behavior of the cell involving aggressive growth, senescence, differentiation and metastasis and/or inflammation is inactivated.

## Description

It has been found that the Caspase-activated DNAse (CAD), an endogenous nuclear enzyme, is frequently activated in human cells, and that it is necessary for a number of biological effects.

The data disclosed in this application show that CAD functions to regulate inflammation, cancer metastasis, senescence and cell differentiation. The present invention relates to the regulation of these biological effects by CAD. It embodies the inhibition of CAD in order to inhibit the above processes therapeutically.

CAD is a DNA-cleaving enzyme that was first described in apoptotic cell death. It turned out, however, that CAD is not essential to apoptosis and recently it has been suggested that CAD can be activated in the absence of cell death. The data, described below, show that CAD can regulate inflammation, cancer metastasis, senescence and differentiation. The inhibition of CAD will therefore inhibit these biological processes and provide a promising therapeutic approach to a number of medical conditions.

During apoptosis, the mitochondrial outer membrane is permeabilized, leading to the release of cytochrome c, which activates downstream caspases. Mitochondrial outer membrane permeabilization (MOMP) has historically been thought to occur synchronously and completely throughout a cell, leading to rapid caspase activation and apoptosis. Newer data show that MOMP does not have to be complete, but that low-intensity, sub-lethal signals can be generated that activate small amounts of caspases and activate CAD (Ichim et al. (2015), Molecular Cell, 57, 860-872).

Role and physiological or pathophysiological effects of this activation of CAD are largely unclear. In the course of the present invention it has been shown that CAD is spontaneously active in human cells, and that it is activated and contributes to biological programs and output in some situations. Thus, CAD facilitates aggressive growth of cancer cells *in vitro* and metastasis in animal models. It was found in the course of this invention that CAD-activity drives a gene expression program that is very strongly linked to unfavorable prognosis in cancer patients. Additional data demonstrate that CAD-activity can contribute to inflammation, senescence and stem cell differentiation. These results identify CAD as an enzyme with a number of unexpected biological effects. The therapeutic inhibition of CAD is expected to have benefits in cancer, inflammation and cellular ageing, and can through blockade of differentiation have beneficial effects.

### CAD in metastasis

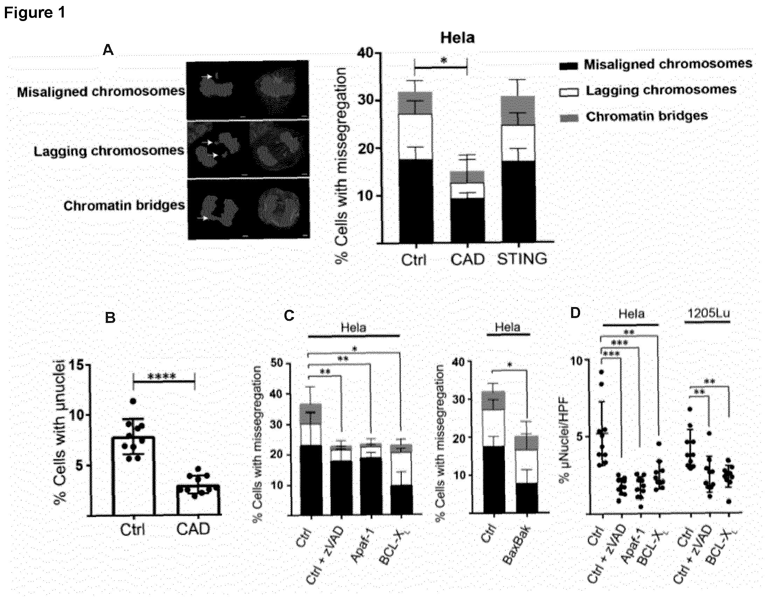

In the course of this invention, it has been found that CAD is spontaneously active in cancer cells, and that in the absence of CAD aggressive growth and metastasis of cancer cells are reduced. Metastasis is linked in the literature to chromosomal instability (CIN), most likely because DNA-ligands are generated that stimulate the cells. We have found that first, CAD is activated in cancer cells *in vitro* through spontaneous activity in the mitochondrial apoptosis system and generates CIN and micronuclei (MN). This is clear because cancer cells modified to lack CAD or components of the (CAD-activating) mitochondrial apoptosis apparatus, or where apoptosis signaling was blocked using a chemical caspase inhibitor (zVAD-fmk), all show reduced CIN (measured as chromosomal missegregation, for instance as chromatin-bridges in dividing cells) and numbers of MN (Fig. 1). All the results shown in this section were obtained with several cell lines; for simplicity, only examples are shown here.

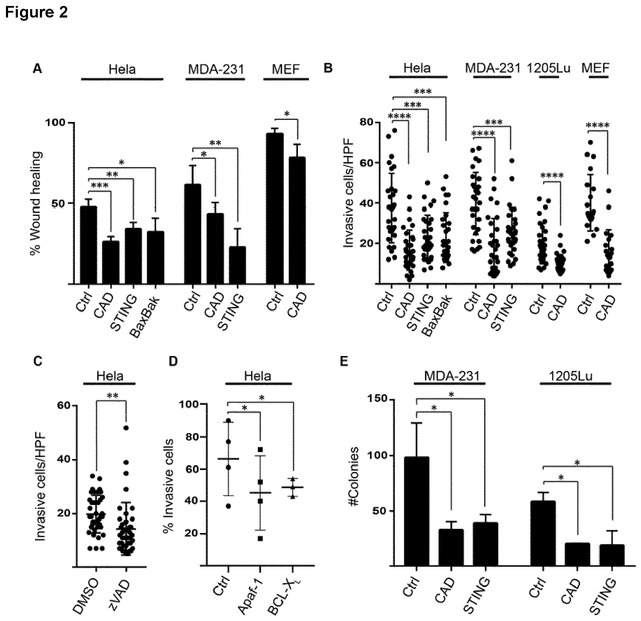

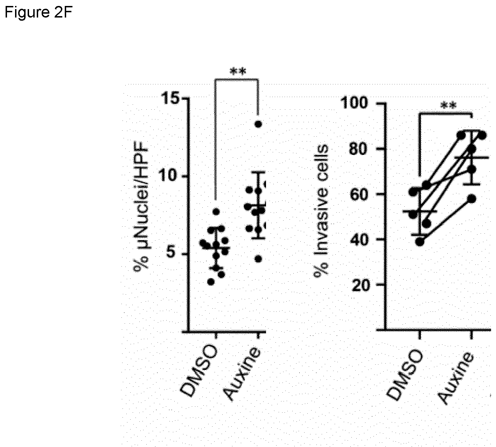

We then investigated the effect of the above modifications on aggressive cancer growth *in vitro*, using the parameter of migration (Fig. 2A), invasion through a basement membrane (Fig. 2B, C) and colony formation on soft agar (Fig. 2C); all these parameters are a measure of aggressive growth in cancer. In all cases, cells lacking either CAD or components of the apoptosis apparatus, or which were inhibited (zVAD, Apaf-1, Bcl-X) in apoptosis (Bcl-X_{L}-overexpression, caspase-inhibition by the pan-caspase-inhibitor zVAD-fmk); showed less aggressive growth. STING-deficient cells are known to show this behavior, and these cells were included as controls. We also used a model where we can directly activate CAD (the model is described in the next section); this was achieved by activating CAD through proteasomal degradation of its inhibitor ICAD. When we activated CAD in this way, the cells contained more micronuclei and became more strongly invasive *in vitro* (Fig. 2F). This confirms that CAD-activity can drive aggressive cell growth.

In two animal models, we observed that CAD-deficient tumour cells metastasized less, showing that CAD is required for metastatic activity.

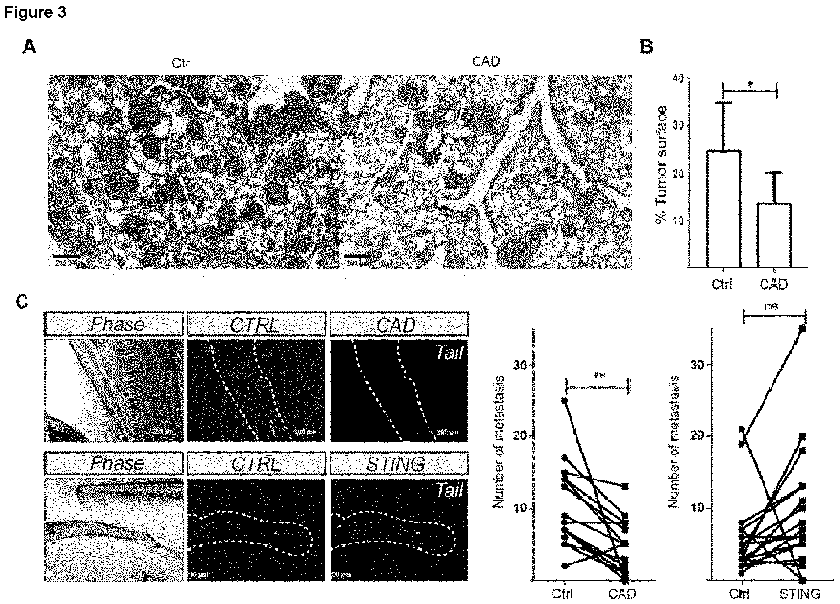

One model we used was a xenograft model of human melanoma cells (the metastatic cell line 1205Lu) into immunocompromised mice. In this model, the melanoma cells are injected into the tail vein of the mice, and they form metastases in the lung (Besch R. et al., J Clin Invest. 2009; 119:2399). We found that melanoma cells engineered to lack CAD caused less of a metastatic burden in the lung (Fig. 3A, B).

In the second model, we used zebrafish, an accepted approach to test for metastatic potential (Cagan R.L. et al., Developmental Cell 2019, 49:317). In this model, cells are injected into the perivitelline cavity of fish larvae, and metastasis into the vessels of the caudal fin is monitored. We infected control and CAD-deficient cells of the human breast carcinoma cell line MDA-MB231 and observed that metastasis was significantly reduced if the cells lacked CAD (Fig. 3C). Thus, CAD is required for the full metastatic activity of cancer cells.

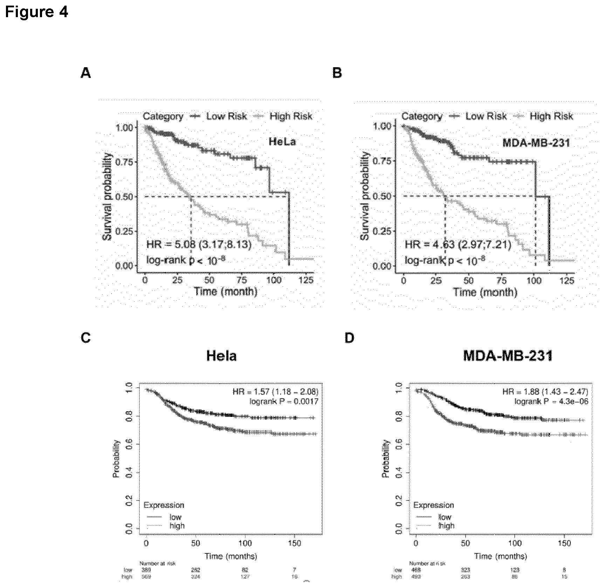

The results suggested that CAD drove gene expression that determined metastatic behavior. RNA-sequencing was conducted and a large number of deregulated genes in cells deficient for CAD was found (comparing control and CAD-deficient cells for two cell lines, HeLa and MDA-MB231, data not shown). We constructed a 'gene-signature' from the most highly or most significantly CAD-regulated genes and tested the relevance of these genes for patient survival using published data of gene expression in cancers where patient outcome had been tested. With very high significance, the high expression of these CAD-dependent genes were associated with poor survival in the patients (Fig. 4). Because most cancer patients die of metastases, this result supports the earlier results that CAD drives metastasis: CAD-activity is significantly associated with the expression of these genes and with metastasis.

The results presented here show that CAD-activity is very clearly associated with chromosomal defects, aggressive growth *in vitro* and metastasis *in vivo*, as abundantly evidenced by the effect of the deletion of CAD or the inhibition of the upstream processes of CAD, as well as the effect of direct CAD-activation. A chemical inhibitor of CAD will block aggressive growth and metastasis in cancer patients and can be used therapeutically to inhibit the progression of cancer, in particular through blocking metastasis.

### CAD in inflammation

Inflammation is a complex process that is initiated upon pathogen recognition and tissue damage, and which serves as the first step in an immune response. Inflammation is a necessary and physiological process. At the same time, excessive inflammation is destructive, and numerous human illnesses are linked to hyperinflammation, such as in allergic and autoimmune disease. In the course of this invention, we have identified CAD as a driver of inflammation as described in this section.

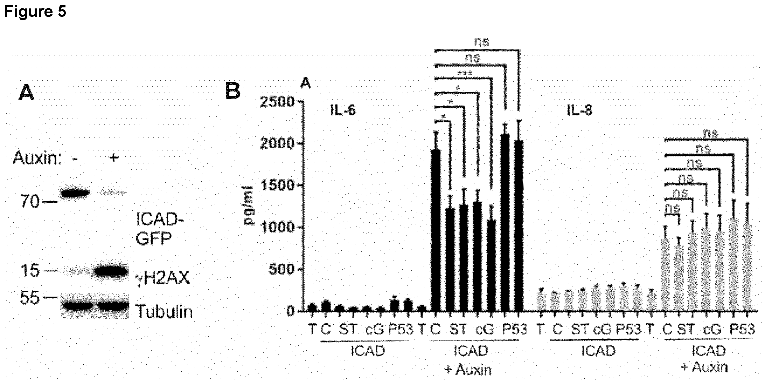

We have established a model where CAD can be activated directly in human cells (already referred to above). The system uses the synthetic biology-concept of the auxin-induced degron, whereby a protein can be destroyed in a targeted fashion by proteasomal degradation upon addition of the plant hormone, auxin. We established three different cell lines (human: HaCaT keratinocytes and Hela; mouse: B16-melanoma) where the inhibitor of CAD, ICAD, can be degraded in this way. When ICAD is degraded, the cells secrete inflammatory chemokines and cytokines (Fig. 5: HaCaT cells; HeLa not shown). We further performed RNA-sequencing experiments in HaCaT cells and found the enhanced activation of numerous pathways associated with host defence and inflammation (not shown). This 'gain-of-function' model therefore shows that CAD, when directly activated; causes a pro-inflammatory activity of human cells, and CAD is sufficient to cause inflammation.

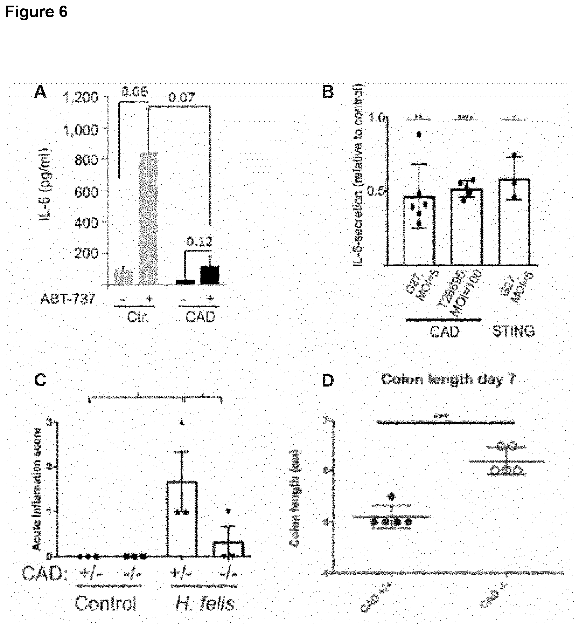

We then tested to see if CAD was also necessary to drive inflammation. In the first model, CAD-deficient HeLa cells were treated with the Bcl-2/Bcl-X_{L}-inhibitor ABT-737, which has been reported to activate CAD and to cause the secretion of inflammatory chemokines and cytokines. As shown in Fig. 6A, CAD-deficient Hela cells made almost no IL-6 (the same was found for IL-8 (not shown)). When CAD-deficient cells were infected with the pro-inflammatory, stomach-dwelling bacterium *Helicobacter pylori*, the secretion of IL-8 was reduced by about half (Fig. 6B). When primary mouse airway epithelial cells were infected with Influenza virus, the secretion of inflammatory cytokines and the induction of pro-inflammatory genes was also reduced (not shown). We infected CAD-deficient mice with *Helicobacter felis* and found significantly reduced inflammation in the stomach (Fig. 6C). We then used the inflammatory model in mice where colitis is induced by adding DSS to the drinking water. CAD-deficient mice had significantly less inflammation in their colon (Fig. 6D). This set of experiments shows that CAD is required for normal inflammatory activity of human cells. Together with the results above (that CAD on its own is pro-inflammatory) the data identify CAD as a critical component of the induction of inflammation. Reduction of expression or inhibition of CAD will therefore have a therapeutic effect in inflammation, and inflammation can be reduced by the inhibition of CAD-activity.

### CAD in senescence

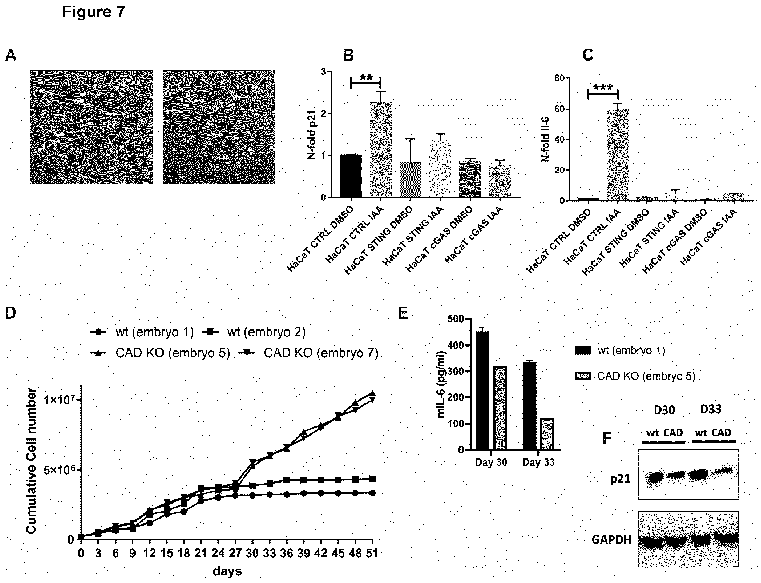

Senescence is a cellular program that is activated during ageing and that is also relevant to tumor development. Senescent cells stop dividing and secrete cytokines; senescence in incipient tumor cells is a mechanism that blocks tumor development. Senescent cells on the other hand secrete soluble factors, which can affect inflammation and tumor progression. Senescence is associated with damage to genomic DNA and a DNA-damage response, but the mechanism of this DNA-damage has been unclear. As shown in the course of this invention, CAD is a key contributor to senescence. We used the model of direct, auxin-mediated CAD-activation in HaCaT cells to show that repeated activation of CAD is sufficient to induce senescence, as measured by the typical senescent morphology (Fig. 7A) and the expression of the typical senescence-associated genes IL-6 and p21 (Fig. 7B, C).

It has been shown that CAD is also necessary for the induction of senescence by testing senescence in a number of models of CAD-deficiency. Mouse embryonic fibroblasts undergo replicative senescence during prolonged culture, which is detectable by a stop in replication. This stop was not observed in fibroblasts isolated from CAD-deficient cells (Fig. 7D). Senescence in these cells was confirmed by the expression of IL-6 and p21, which differed substantially between wt and CAD-deficient cells (Fig. 7E, F). CAD was also required for oncogene (oncogenic ras)-induced senescence in fibroblasts and for senescence induced by the drugs Palbociclib and ABT-737 in human cancer cells (not shown). *In vivo*, aged CAD-mice also showed less signs of cellular senescence. CAD is therefore a critical mediator of cellular senescence, and its inhibition is a promising strategy to reduce senescence and perhaps ageing.

### CAD in cellular differentiation

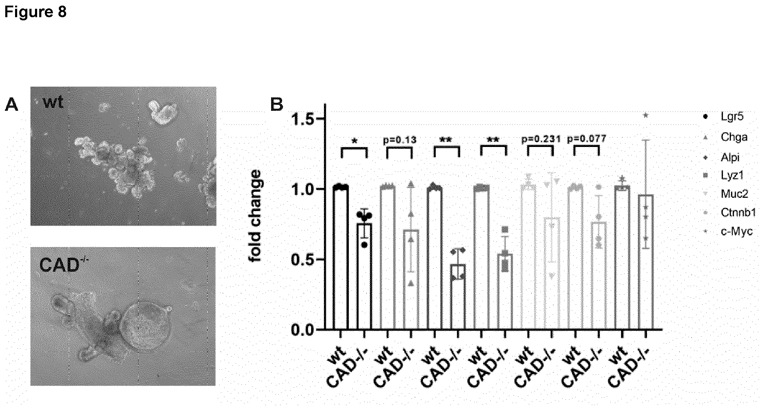

Tissue cells differentiate from stem cells through specialized gene expression programmes. As shown in the course of this invention, mouse intestinal organoids do not differentiate normally if they lack CAD, linked to a lack of the normal changes in gene expression. CAD is therefore required for differentiation, and inhibition of CAD may block differentiation. Fig. 8A shows the morphology of small intestinal organoids that were generated from primary cells of wt or CAD-deficient mice. Note the lack of differentiation in CAD-deficient organoids. When the expression of genes that accompany the differentiation in these organoids was investigated, we noticed that differentiation-associated genes were less expressed in organoids from CAD-deficient mice (Fig. 8B), confirming that these organoids fail to undergo differentiation.

The present invention relates to a method of preventing or reducing metastasis of tumor cells wherein the Caspase-Activated DNAse is inactivated wherein the Caspase-Activated DNAse induces strand breaks in the cell's genomic DNA in the absence of apoptotic cell death. It further relates to a method of reducing inflammation, when CAD is activated and induces inflammatory effects. It further relates to a method reducing cellular senescence, where the occurrence of senescence is associated with CAD-activity. Finally, it relates to a method reducing differentiation of human cells where differentiation receives a contribution from CAD-activity. The method involves reducing the activity of CAD, with the aim to reduce the aggressive growth of cancer cells (including a reduction of metastasis), to reduce inflammation, to reduce senescence or to inhibit differentiation.

There are several possible ways to inactivate the caspase-activated DNAse or to prevent its activation. CAD is activated by caspase-proteases. Caspases are always present in a cell but become activated during either apoptosis or during sub-lethal signaling in the apoptosis apparatus. CAD is co-translated with its inhibitor ICAD, which serves as an essential chaperone (without ICAD, no CAD can be produced). During activation of CAD, ICAD is cleaved by caspases at specific sites of the protein. If this cleavage is prevented, CAD-activation is blocked. CAD-activity can be prevented by disrupting this process. Alternatively, CAD-activity can be reduced or blocked by directly targeting CAD.

In further embodiments the caspase-activated DNAse is directly inhibited by chemical molecules. Such molecules can be identified by testing their effect on the activity of CAD either in intact mammalian cells or in a cell-free system, where individual components (such as cell lysates or recombinant proteins) are used.

In another embodiment, the degradation of CAD is induced by the use of a chemical CAD-binding molecule, which directs the proteasomal degradation of CAD.

In intact cells, CAD-activity can be generated by either inducing apoptosis or by inducing sub-lethal apoptosis signaling. A further method is to express a modified inhibitor of CAD (ICAD) in cells where endogenous ICAD has been deleted. This modified inhibitor contains an auxin-induced degron and can be degraded by addition of the small molecule auxin. The activity of CAD can then be measured by assessing cleavage of/damage to genomic DNA (for instance by Comet assay), by detecting a DNA-damage response (for instance by detecting the phosphorylation of the histone H2AX (yH2AX)) or by detecting the induction of a CAD-regulated gene.

In cell-free systems, CAD-activity can be generated by preparing extracts from cells undergoing apoptosis or producing sub-lethal apoptosis signals. It can also be generated by using recombinant caspase-3, which can be added to cell extracts or to a recombinant ICAD/CAD-complex.

In all these situations, active CAD is generated, and its activity can be measured by a variety of assays (for instance adding DNA and measuring its degradation on an agarose gel). Potential inhibitors are added to intact cells or to the cell-free system, and the effect on CAD-activity is recorded.

Another way of preventing or blocking the activity of CAD is the inhibition of activation and/or activity of the enzyme by expressing a mutant version of ICAD (where the cleavage by caspases has been disabled). Such a construct can be introduced as a DNA/RNA-construct coding for the protein (or fragments thereof), or as a cell-permeable protein/peptide and delivered by various means to patients.

Another way of preventing CAD-activity is the blockade of caspases with chemical inhibitors. Because caspase-activity is required for the activation of CAD at least in most situations, this will also prevent the downstream effects of CAD in terms of tumor growth/progression and of inflammation.

Another embodiment to inhibit the caspase-activated DNAse is to inhibit the expression of CAD or of ICAD by RNAi-methods or by deleting/modifying the genes (such as by CRISPR/Cas), blocking their expression as an intact protein. In this embodiment, the constructs are applied to patients by various routes (for instance intravenously, intratumorally, or by other means of systemic or local application).

The methods of the present invention may be used for treating subjects and are in particular performed *in vitro.* This can be done for example by isolating suitable cells from a patient to be treated and to genetically modify them. Such cells which produce gene products acting as inhibitor of the caspase-activated DNAse are produced by such transformed cells. Such cells are proliferated and expanded *in vitro* and may thereafter be applied to human beings. Alternatively modified genes contained within a suitable vector are administered to the patient to be treated.

In another embodiment the caspase-activated DNAse is inhibited by small chemical molecules which may be administered to the subject to be treated in a suitable pharmaceutical formulation. Suitable molecules can be identified by an *in vitro* method whereby a test system is established wherein the normal activity of the caspase-activated DNAse is measured. For example, extracts can be prepared from apoptotic cells and incubated with isolated nuclei or other sources of DNA. Extracts can also be prepared from non-apoptotic cells and activated using recombinant caspase. It is also possible to use recombinant CAD-ICAD protein complex and recombinant caspase-3. In all these approaches, active CAD is generated, which then degrades the DNA, which can be measured for instance by agarose gel electrophoresis. Then potentially suitable molecules can be added and their inhibitory effect can be measured.

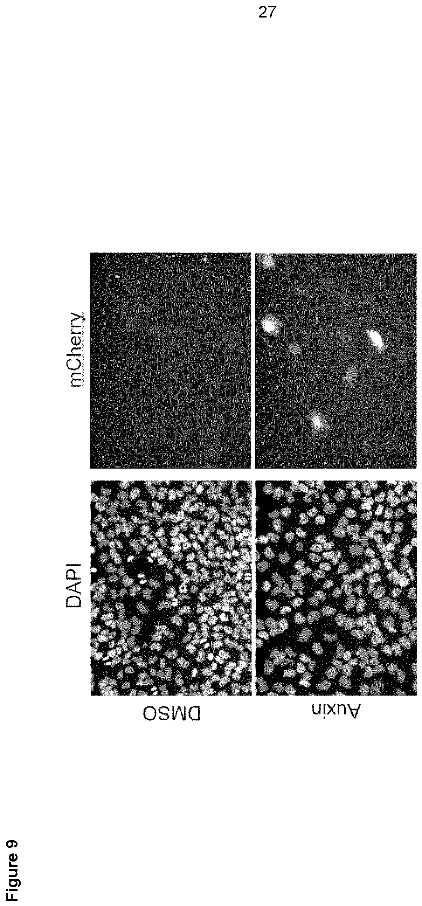

The results of the experiments which show preferred embodiments of the present invention have been summarized in the Figures and experiments. We are using a screening system where we have used the cells were auxin activates CAD as described above (background: Hela cells). Because we had observed that the cells make interleukin 8 (IL-8) when CAD is activated, we generated a 'knock-in' cell line where the gene for the fluorescence protein mCherry was placed in the IL-8-locus. When CAD is activated by auxin (and the IL-8 promoter is induced), the cells show mCherry-fluorescence of varying strength (see Fig. 9). We use these cells, add candidate inhibitors from a chemical library, and test by microscopy (as in Fig. 9) whether the inhibitor blocks the appearance of mCherry-positive (fluorescent) cells. Substances that do block this will be investigated further to confirm their CAD-inhibitory activity.

In summary, the data identify a role for sub-lethally activated CAD in regulating cancer cell aggressive growth and metastasis, inflammation, senescence and cellular differentiation. In cancer cells, CAD generates a phenotype that has features of epithelial-mesenchymal transition, that supports metastasis and that is linked to poor prognosis in cancer patients. CAD appears to be an attractive drug target for the therapeutic inhibition of cancer progression. The data also identify a role of CAD in triggering and maintaining inflammation. CAD appears to be an attractive target to reduce inflammatory disease. The data further show that CAD is both required and sufficient for the induction of senescence. CAD-inhibition is therefore an attractive approach to prevent or reduce senescence. Lastly, the data show that CAD plays a role in cellular differentiation. Inhibition of CAD is therefore an attractive approach to modify differentiation.

In the present application the following abbreviations were used:

**Table 1**

| | |
|---|---|
| CAD | Caspase-Activated DNAse |
| CIN | Chromosomal Instability |
| Ctrl | control cell line |
| cGAS | cyclic GMP-AMP Synthase |
| DDR | DNA-Damage Response |
| ICAD | Inhibitor of Caspase-Activated DNAse |
| HeLa | cervical carcinoma cell line |
| MDA-MB-231 | breast carcinoma cell line |
| 1205 Lu | metastatic melanoma cell line |
| STING | Stimulator of Interferon Genes |
| WT | Wild Type |

The invention as disclosed in the general part of the specification is more specifically disclosed in the experiments and Figures.

### Figure legends

### Fig. 1 Spontaneous CAD-activity drives chromosomal missegregation and the appearance of micronuclei

**A**, the various pictures of chromosomal missegregation/misalignment are shown as examples (left) and enumerated in HeLa cells (cervical carcinoma line; ctrl., control; CAD, lacking CAD; STING, lacking STING; STING is not relevant to this invention).
**B**, numbers of micronuclei in cells with (control) or without CAD (MDA-MB-231 mammary tumour cells). Results are given as percentage of cells containing micronuclei.
**C**, missegregation in HeLa cells with inhibition in the mitochondrial apoptosis apparatus. Symbols as in A. zVAD-fmk is a small molecule pan-caspase-inhibitor. Apaf-1 is an adapter protein of caspase-9 that is required for caspase-activation upon release of cytochrome c from mitochondria. Both Bax and Bak can directly release cytochrome c, and the presence of at least one of them is required for mitochondrial apoptosis. Bcl-X_{L} is a mitochondrial protein that blocks the activation and activity of Bax and Bak and thereby inhibits apoptosis.
**D**, micronuclei in HeLa cells or 1205Lu cells (metastatic melanoma cells) with inhibition in the mitochondrial apoptosis apparatus. Results are given as percentage of cells containing micronuclei.

Different cell lines were used that were either control (denoted Ctrl.; carrying a non-coding gRNA) or deficient in CAD, Apaf-1 or both Bax/Bak (CRISPR/Cas-deletions). Bcl-X_{L}, cells over-expressing the apoptosis inhibitor Bcl-X_{L}. Apaf-1 and Bax/Bak are proteins required for mitochondrial apoptosis. Bcl-X_{L}-overexpression blocks mitochondrial apoptosis. Ctr. + zVAD: cells were incubated for one day with the caspase-inhibitor zVAD-fmk. Throughout these legends, cells lacking an individual gene are denoted by the name of the encoded protein. These results illustrate two points. First, they show that CAD is always active in tumour cells. Secondly, because chromosomal missegregation and micronuclei are known to be linked to metastasis, these results suggest that CAD-activity through these events contributes to metastasis.

### Fig. 2 Altered growth characteristics in CAD-deficient tumor cells

Various parameters of aggressive cell growth *in vitro* were measured:
**A**, migratory activity of cells with or without CAD (scratch assay). Percentages of the area closed upon scratch are given. In this assay, a monolayer of cells is mechanically scratched, and the cells migrate back to fill the gap. The speed with which they migrate is a measure of their migratory capacity.
**B**, **C** invasion through a basement membrane (number of cells are given that had invaded).
**D**, colony formation in soft agar (number of colonies). The invasion assay uses a basement membrane in a culture dish. The cells are seeded on top of the membrane, and after a predetermined time (here: 24 h), the number of cells at the bottom of the membrane is determined. This is a measure of the capacity of the cells to invade tissue. Colony formation measures a capacity also termed 'anchorage-independent growth'. It is a particular capacity of metastatic cells to be able to grow to colonies without support from other cells of solid support (as in a plastic dish). Individual cells are seeded in soft agar to immobilize them and to test whether the single cells can grow and form colonies, a feature of metastatic cells. The number of colonies formed is then measured.

The cell lines and conditions were described in the legend to Fig. 1. zVAD, caspase-inhibitor zVAD-fmk. Ctrl., control cells; the other cell lines are labelled according to their genetic modification (gene deletion; Bcl-X_{L}-overexpression). MEF, SV40-transformed mouse embryonic fibroblasts from wt or CAD-deficient mice.

**F**, HaCaT cells engineered to express active CAD upon addition of the plant hormone auxin contain more micronuclei and are more invasive. Left, cells were treated with solvent (DMSO) or auxin (20 µM; to activate CAD) for 6h. Auxin was washed out, and culture was continued for 24h. Cells were stained with DAPI and beta-tubulin and assessed by microscopy for micronuclei. A minimum of 10,000 cells per group were counted. Symbols give the percentages of micronucleus-positive cells per high power field. Columns/error bars are mean /SD of 10 high power fields. **, p<0.01. Note the higher number of micronuclei when CAD had been activated. Right, HaCaT were treated with auxin (20 µM) and tested for invasion through a Matrigel basement matrix. The percentage of cells that had migrated after 24 h is shown. Data shown are means/SD of 4-5 independent experiments. **, p<0.01. Note the higher number of invasive cells upon CAD-activation by auxin (auxine).

### Fig. 3 Reduced metastasis of CAD-deficient tumor cells in two animal models

Control or CAD-deficient tumor cells were injected into the tail veins of immunocompromised mice (1205Lu human melanoma cells; **A, B**) or into the perivitelline cavity of zebrafish larvae (MDA-MB231 mammary carcinoma cells; **C**). **A, B**: mice were sacrificed after 15 days, and metastases in the lungs were measured by histology (**A**, H&E-stain; **B**, quantification of data from six mice). In this model, if cells are metastatic they migrate into the lung tissue and form metastases. **C**, MDA-MB-231 human breast cancer cells (carrying a non-coding gRNA (CTRL) or deficient in CAD or STING were injected (about 300 cells per larva). In this model, metastatic cells are able to migrate into the blood vessels and are found in the caudal fin, where they can be counted by microscopy. Cells metastasized to the caudal fin were counted (left, original pictures as examples; right, quantification). **C**, CAD-deficiency but not STING-deficiency reduced metastasis.

### Fig. 4 Expression of CAD-regulated genes is associated with poor prognosis in cancer patients

The prognostic impact of CAD deficient gene signatures on survival of patients from the TCGA data collection was examined using the Cox proportional hazard model. The TCGA-data are publicly available gene expression data from tumours of patients. We used the CAD-signature as a measure of CAD activity and stratified the patients for high or low CAD-activity based on this expression. In the same database, the history of the patients is also recorded, permitting to align CAD-activity (i.e. gene signature) with survival. The CAD-signatures from HeLa cells (comparing RNA-sequencing results in control and CAD-deficient cells) and from MDA-MB231 cells were different, and we therefore tested them separately. The Cox proportional hazard model is a statistical model that is commonly used to measure associations (here gene signature) with patient outcome. Association of a CAD-signature derived from Hela (**A**) and MDA-MB-231 (**B**) with survival. Signatures comprised of genes differentially expressed between control and CAD-deficient cells (padj<0.01). Patients were assigned to high- vs. low-risk groups based on their expression levels of the signature genes in tumour vs. normal tissue. In all cases, a significant separation (log-rank p-value < 10⁻⁸) between high and low risk subjects is achieved. The results show that patients with evidence of high CAD-activity (high expression of CAD-regulated genes) have a higher risk of death, suggesting that CAD drives metastasis and death in these patients.

**C-D** Distant metastasis-free survival in a cohort of 958 breast cancer patients stratified according to the expression of the 10 most downregulated genes in each cell line. The gene (mRNA-) expression data are from a published study that measured gene expression in breast cancer patients (Gyorffy B et al., Breast Cancer Res Treat 2010, 123:725). We stratified these patients into groups with high or low CAD-activity (using the 10 most strongly CAD-dependent genes based on our RNA-sequencing data from either HeLa of MDA-MB231 cells) and plotted patient survival of the two groups. Note that patients whose tumour showed this evidence of higher CAD-activity had poorer survival, suggesting that the CAD-dependent driving of metastasis occurs more frequently in these patients.

### Fig. 5 Inflammatory activity induced by CAD-activation in HaCaT human keratinocytes

**A,** loss of ICAD-AID-GFP and induction of a DNA-damage response (yH2AX-signal indicates a DDR) upon treatment of HaCaT cells engineered to respond to auxin with the proteasomal degradation of ICAD (cells were treated with auxin or not for 6 h, 20 µM). Note the loss of ICAD and the appearance of the yH2AX-signal, indicating CAD-activation.
**B**, Chemokine/cytokine response to CAD-activation in HaCaT cells. Secretion of IL-6 or IL-8 was measured by ELISA upon treatment of HaCaT TIR-only (control cells where CAD is not activated by auxin) or HaCaT-ICAD cells (where auxin induces CAD-activation, see A). An increase of IL-6 and/or IL-8 is an indicator of inflammatory reactions. The same cells deficient in STING (labelled ST), cGAS (cG) or p53 were included to understand the signalling pathways; this is irrelevant to the present invention) Treatment was with auxin (20 µM for 6 h, when it was washed out and replaced by complete medium without auxin; analysis was done 18 h later). T, TIR-only control cells which do not respond; C, cells responding to auxin with CAD-activation. Auxin indicates treatment; the other cells were treated with DMSO as a control. Left, IL-6, right, IL-8. All values are means/SEM of three independent experiments.

### Fig. 6 CAD is required for inflammation

**A**, *IL-6-induction by sub-lethal apoptosis signaling during ABT-737-treatment requires CAD.*

HeLa cells (control cells or CAD-deficient cells (*18*)) were treated for 72 h with 10 µM ABT-737. IL-6-concentrations in the supernatants were determined by ELISA. Data are means/SEM of three independent experiments. Numbers give significance levels (p-values).

**B**, *the inflammatory reaction to* Helicobacter pylori in vitro *requires CAD.*

HeLa cells (control cells or cells lacking CAD or STING as indicated) were infected with *H*. *pylori* strains G27 or T26695 as indicated. IL-6 was measured in supernatants 24 h post-infection. Values were normalized to the amounts measured in supernatants from infected control cells in the same experiments. Each symbol represents one experiment. Columns are means/SEM. *, p<0.05, **, p<0.01, ****, p<0.0001 (for difference to infected control cells (control cells were set to the value of 1; CAD or STING-deficient cells produced roughly 50 % IL-6 of control cells)). The STING-data are not relevant for the invention but are a useful comparison as STING is an established mediator of inflammation.

**C**, **D** In vivo *role of CAD in inflammation.* **C**, *Helicobacter*-infection. Mice of the indicated genotype (CAD heterozygote [indicated as +/-; controls expressing CAD] or homozygote [-/-] CAD-deficient) were infected by oral gavage with *Helicobacter felis* three times (2 days apart each; standard protocol). After seven weeks, mice were sacrificed, gastric sections stained with H&E, and the inflammatory infiltrate was assessed by a trained pathologist (PD Dr. Aumann, University Medical Center Freiburg) (0=normal; 1=mild gastritis; 2=moderate gastritis; 3= marked gastritis). Each data point shows one mouse (*p<0.05; one-way ANOVA). **D**, CAD in DSS-colitis. Colon length as a key measure of inflammatory activity during dextran sulfate sodium-induced colitis (DSS-colitis) in wt (CAD+/+) and in CAD-deficient mice was investigated. Mice were treated for five days with 2.2 % DSS in their drinking water. Cohorts were sacrificed and colon lengths were measured after 7 (left) days of the experiment. *, p<0.05, ***, p<0.001. Each symbol represents one mouse.

### Fig. 7 CAD is critical for senescence

**A-C**, CAD-activation is sufficient to induce senescence. CAD was activated in HaCaT human keratinocytes with auxin (six times-treatment over the course of two weeks). Cells were analyzed by microscopy (**A**) or for gene expression by qPCR (**B**, **C**). **A**, arrows indicate morphologically senescent cells. The induction of the senescence markers p21 and IL-6 in different cells is shown in **B and C.** Senescence may also depend on the signaling proteins STING and cGAS which is, however, not relevant for this invention.

**D-F**, *CAD is required for replicative senescence.* Mouse embryonic fibroblasts from wild type (wt) or CAD-deficient mice were cultured and replated every three days. **D**, cell number was assessed by counting and calculating the absolute number. Wt cells stop dividing and enter a plateau due to senescence, while CAD-deficient cells keep dividing, increasing in number. **E**, IL-6-secretion is a measure of senescence; IL-6 was found to be less in supernatants from CAD-deficient cells, harvested from the cultures in D on the indicated days. **F**, p21-upregulation is a hallmark of senescence, and was found to be reduced in CAD-deficient cells (cells from D, analyzed on the days indicated) (Western blot).

### Figure 8 CAD is required for differentiation of intestinal organoids

Wild type or CAD-deficient (from gene-deficient mice) mouse small intestinal organoids were induced to undergo differentiation *in vitro.* **A**, microscopy shows differentiation in wild type (wt) but much less in CAD-deficient cells. **B**, expression of differentiation-associated genes on day 5 of differentiation (genes investigated are shown on the right). The values are fold-change normalized to wt cells. Note the reduction of gene expression in CAD-deficient organoids.

### Figure 9 A reporter system for CAD-activity to screen for inhibitors

HeLa cells where CAD can be activated by auxin were modified by introduction of mCherry into the interleukin 8 (IL-8) genomic locus. Because IL-8 is made when CAD is active, these cells express the fluorescence mCherry protein when CAD is activated. Top, DMSO-treated cells (solvent control). Auxin, auxin-stimulation to activate CAD. Cells were stained with Hoechst to visualize nuclei (left) and analyzed for mCherry fluorescence (right; microscopic images). More positive cells can be observed at different settings of the microscope.

## Claims

1. Method of influencing the development of cells wherein the caspase-activated DNAse which induces strand breaks in the cell's genomic DNA causing changes of the behavior of the cell is inactivated.

2. Method according to claim 1 wherein the inactivation of the caspase-activated DNAse is caused by an inhibitor of the caspase-activated DNAse.

3. Method according to claim 2 wherein the caspase-activated DNAse is kept inactive by binding to the inhibitor of the caspase-activated DNAse.

4. Method according to claim 3 wherein the cleavage of the inhibitor of the caspase-activated DNAse is inhibited.

5. Method according to claim 1 **characterized in that** the caspase-activated DNAse is inactivated by a modification with the CRISPR/Cas system.

6. Method according to claim 1 **characterized in that** the expression of caspase-activated DNAse is inhibited by suitable nucleic acids.

7. Method according to any of claims 1 to 6 **characterized in that** the influencing of the development of cells is the prevention or reduction of metastasis of tumor cells which changes the behavior of the cells, in particular aggressive growth and metastases.

8. Method according to any of claims 1 to 6 wherein the influencing of the development of cells is the prevention or reduction of inflammation.

9. Method according to any of claims 1 to 6 wherein the influencing of the development of cells is the inhibition or reduction of senescence and ageing.

10. Method according to any of the preceding claims wherein the method is performed *in vitro.*

11. Method according to any of claims 1-10 **characterized in that** the caspase-activated DNAse is inhibited by a chemical molecule.

12. Method according to any of claims 1-10 **characterized in that** an inhibitor of the caspase-activated DNAse is encoded by a vector whereby the inhibitor is expressed therefrom.

13. Method of identifying molecules which inhibit the caspase-activated DNAse by performing an *in vitro* test system whereby the inactivating activity of a compound is measured by contacting said compound with the caspase-activated DNAse in an *in vitro* test system comprising the preparation of extracts containing CAD-activity or a system where CAD-activity is generated by the use of recombinant components and adding thereto recombinant caspase and the compound to be tested.

14. Method according to claim 13 wherein the test system comprises the use of immunocompromised mice.
